(19) Europäisches Patentamt
European Patent Office
Office européen des brevets

(11) Publication number: **0 021 688**
**B1**

(12) # EUROPEAN PATENT SPECIFICATION

(45) Date of publication of patent specification: **13.07.83**

(21) Application number: **80301917.3**

(22) Date of filing: **06.06.80**

(51) Int. Cl.[3]: **C 07 D 501/20,**
**A 61 K 31/545**
**//C07D277/42**

(54) **7-(Thiazolylpropionamido) cephalosporins, their preparation, and pharmaceutical compositions containing them.**

(30) Priority: **07.06.79 JP 71595/79**
**10.03.80 JP 30019/80**

(43) Date of publication of application:
**07.01.81 Bulletin 81/1**

(45) Publication of the grant of the patent:
**13.07.83 Bulletin 83/28**

(84) Designated Contracting States:
**DE FR GB IT**

(56) References cited:
**EP - A - 0 001 125**
**FR - A - 2 343 747**
**FR - A - 2 356 654**

(73) Proprietor: **YAMANOUCHI PHARMACEUTICAL CO. LTD.**
**No. 5-1 Nihonbashi-Honcho, 2-chome Chuo-ku**
**Tokyo (JP)**

(72) Inventor: **Iwanami, Masaru**
**No. 8-1 Nishigaoka**
**Kohoku-ku Yokohama-shi (JP)**
Inventor: **Koda, Akio**
**No. 3-10-13, Shimohoya Hoya-shi**
**Tokyo (JP)**
Inventor: **Murakami, Yukiyasu**
**No. 306-3 Omaki Urawa-shi**
**Saitama (JP)**

(74) Representative: **Geering, Keith Edwin et al,**
**REDDIE & GROSE 16 Theobalds Road**
**London WC1X 8PL (GB)**

**EP 0 021 688 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England

7-(Thiazolylpropionamido) cephalosporins their preparation, and pharmaceutical compositions containing them

This invention relates to cephalosporins wherein a propionamido group having a free or substituted hydroxy group at the 3-position and a thiazolyl group at the 2-position is linked to the 7-position of the cephalosporin and to their production.

Various kinds of cephalosporin derivative are known, and cephalosporins having as a side chain acylamino group at the 7-position a propionamido group in which the 3-position is substituted with a hydroxy group and the 2-position is substituted with a heterocyclic group are disclosed in Japanese Patent Applications (Open for Public Inspection) Nos. 101,338/'78, 16,495/'79 and 36,286/'79. These cephalosporins show some antibacterial activity against Bacillus subtilis which is a gram positive bacterium, but little against species such as Shigella sonnei and Proteus mirabilis.

The present invention provides cephalosporin derivatives represented by following general formula I

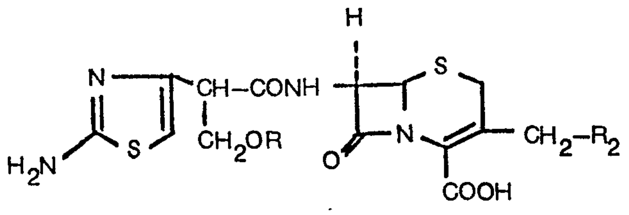

I

wherein

R represents hydrogen or a carbamoyl group and $R_2$ represents an acetoxy group, a (1-methyltetrazol-5-yl)thio group or a (5-methyl-1,3,4-thiadiazol-2-yl)thio group, and optionally one or both of the amino group and the carboxylic acid group is protected; and salts thereof.

French Specification No. 2,343,747 discloses various antibacterial cephalosporin derivatives including, for example, 3-(1-methyl-1H-tetrazol-5-yl)thiomethyl-7-[2-(2-amino-1,3-thiazol-4-yl)glyoxylamido]-3-cephem-4-carboxylic acid, but antibacterial activity is demonstrated only against E. coli. French Specification No. 2,356,654 also discloses various antibacterial cephalosporin derivatives, including for example 2-methyl-7-[2-hydroxy-2-(2-aminothiazol-4-yl)acetamido]-3-cephem-4-carboxylic acid, but these differ from the compounds of the present invention in being substituted at the 2-position rather than the 3-position of the cephalosporanic ring. Antibacterial cephalosporin compounds disclosed in European Specification No. 0,001,125 also differ structurally from those of the present invention, e.g. in the nature of the substituent at the 3-position and of the amido group at the 7-position.

As a protective group for the amino group, one conventionally used in the fields of cephalosporin and peptide chemistry is preferably employed, and examples of such protective groups are the trityl, trimethylsilyl, formyl, propionyl, methoxyacetyl, benzyloxy, carbonyl, t-butoxycarbonyl and phthaloyl groups. As a protective group for the carboxy (i.e. carboxylic acid) group, one conventionally used in the above-mentioned fields is preferably employed, examples being the methyl, trimethylsilyl, $\beta$-methylsulfonylethyl, phenacyl, *p*-methoxybenzyl, nitrobenzyl, benzyl, benzhydryl, *t*-butyl, phthalidyl and pivaloyloxymethyl groups.

The protective groups used are preferably those which, when the compound is administered to a living body, accelerate absorption of the compound and are easily released in the body. Examples of such protective groups are acetyl and carboxy groups for the amino group, and phthalidyl and pivaloyloxymethyl groups for the carboxylic acid group.

The cephalosporin compounds of this invention show antibacterial activity against various pathogens, particularly such as Shigella sonnei and Proteus mirabilis. They may be made up in a carrier to provide pharmaceutical compositions. The antibacterial activities of compounds of formula I are shown in the following Table, in comparison with known compounds having similar structures.

TABLE

(antibacterial activity: minimum inhibiting concentration $\mu g/ml$)

| Microorganism | Compound of this invention Ex. 2 | Ex. 3 | Ex. 4 | Ex. 5 | Known compound A | B |
|---|---|---|---|---|---|---|
| Shigella sonnei | 0.39 | 0.78 | 1.56 | 0.39 | 3.13 | – |
| Proteus mirabilis | ≤0.2 | 1.56 | ≤0.2 | ≤0.2 | – | 25 |

Compound A:

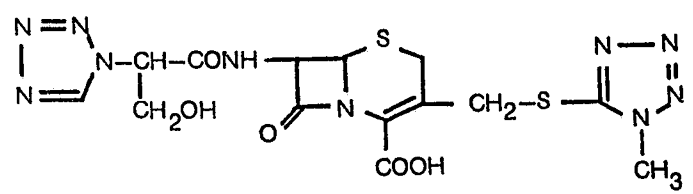

(Japanese published unexamined Pat. appln. No. 101388/1978)

Compound B:

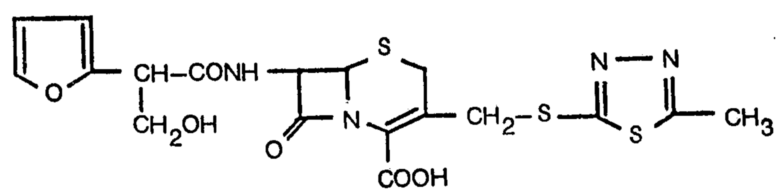

(Japanese published unexamined Pat. appln. No. 36286/1979)

The cephalosporin compounds of formula I can be administered orally or parenterally, in their free form or in the form of salts, for the treatment of diseases of human beings and animals. The dosage of the cephalosporin compounds depends *inter alia* on the condition, weight, and age of the patient but for an adult human is usually a total of 250—3000 mg per day, administered in 3 to 4 doses spaced over the day.

The forms of the compounds of the invention suitable for administration include injections, tablets, capsules, syrups, etc., and can be prepared conventionally using excipients, preservatives, stabilisers, etc., commonly used in medicament formulations.

A cephalosporin compound of formula I can be prepared by reacting a 7$\beta$-aminocephalosporin derivative shown by general formula II

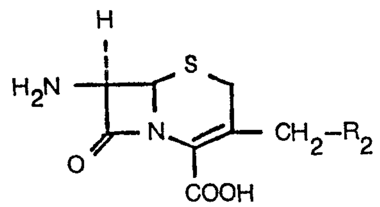

or a reactive derivative thereof in which the amino group is substituted, wherein $R_2$ has the same significance as above and the carboxy group may be protected by a conventional protective group, with (b) a thiazolylpropionic acid derivative represented by the general formula

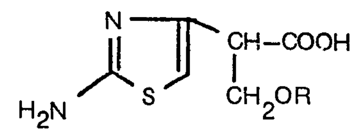

III

or a reactive derivative thereof in which the carboxylic acid group is substituted, wherein R has the same significance as above and the hydroxy group when R is hydrogen and/or the amino group may be protected by a conventional protective group, if desired treating the product to convert any protected group to the free group, and optionally converting the reaction product to a salt thereof.

In the cephalosporin derivative of formula II used as a raw material in the above preparation the carboxy group at the 4-position may be in the form of its sodium, potassium or triethylamine, etc. salt.

The reaction of the compound of formula II and the compound of formula III is suitably performed in a solvent with cooling or at room temperature. Any solvent which does not adversely influence the reaction may be used; suitably one or more of tetrahydrofuran, acetone, chloroform, methanol, ethanol, methylene chloride, ethylene chloride, acetonitrile, ethyl acetate, ethyl formate, and dimethylformamide is used.

Suitable reactive derivatives of the formula III compounds in which the carboxy group is substituted include acid halides, mixed acid anhydrides, active esters, active amides, acid anhydrides, acid azides, etc.

When the formula III compound is an acid halide such as an acid chloride or is an acid anhydride or mixed acid anhydride, it is preferred to perform the reaction in the presence of a base. Examples of suitable bases are organic bases such as triethylamine, pyridine, dimethylaniline, etc., and inorganic bases such as an alkali carbonate, an alkali hydrogencarbonate, etc.

When the formula II compound has a free amino group and the formula III compound a free carboxy group it is better to use a condensing agent such as N,N′-cyclohexylcarbodiimide, N,N′-diethylcarbodiimide, etc.

In the case when R is hydrogen, the hydroxy group may, if necessary, be protected by a protective group conventionally used in the field of peptide chemistry, e.g. an acetyl group; the protective group can be released by treating with a weak base.

A compound of formula I with a free amino group and/or a free carboxy group can be prepared from a corresponding protected compound by releasing the protective group or groups in conventional manner. For example, a protective group for the amino group, such as a trityl or *t*-butoxycarbonyl group, can be released by an acid such as trifluoroacetic acid, and a protective group such as a *p*-nitrobenzyloxycarbonyl group can be released by catalytic reduction. A protective group for the carboxy group, such as a benzhydryl or *p*-methoxybenzyl group, can be released by an acid and a protective group such as a trimethylsilyl group can be released by the contact with water.

The compounds of formula I with a free carboxy group can be converted to pharmaceutically acceptable non-toxic salts thereof in conventional manner. For example, an alkali metal salt of the compound is obtainable by adding to the compound a *n*-butanol solution of an alkali metal 2-ethylhexanoate and then an organic solvent having a different solubility, such as ether, ethyl acetate, etc.; an organic base salt is obtainable by adding thereto an equivalent or slightly excess amount of an

organic base such as dicyclohexylamine, triethylamine, diethanolamine, arginine, lysine, etc.; and an ammonium salt is obtainable by adding aqueous ammonia. The compounds of formula I wherein with a free amino group can also be converted into the pharmaceutically acceptable non-toxic salts thereof in conventional manner, e.g. by adding to the compound an equivalent or slightly excess amount of an inorganic acid such as hydrochloric acid, phosphoric acid, sulfuric acid, etc., or of an organic acid such as fumaric acid, maleic acid, malic acid, citric acid, benzoic acid, etc.

Reference Example 1

In 20 ml of dimethylsulfoxide was dissolved 4.14 g (0.01 mole) of methyl 2-tritylaminothiazol-4-yl acetate and after adding to the solution 413 mg of paraformaldehyde (80% purity) and then 30 mg of sodium methoxide, the mixture was stirred for 4 hours at room temperature.

To the reaction mixture was added 100 ml of ice-water and after neutralizing it by adding a small amount of hydrochloric acid, the reaction mixture was extracted once each with 50 ml and 30 ml of ethyl acetate. The ethyl acetate extract was washed four times with water and after drying with anhydrous magnesium sulfate, the solvent was distilled off to provide 3.5 g of crude methyl 3-hydroxy-2-(2-tritylaminothiazol-4-yl)propionate. The product was purified by silica gel column chromatography using a mixture of benzene and ethyl acetate in 3:1 by volume ratio as eluent.

(i) Elemental analysis for $C_{26}H_{24}N_2O_3S.\frac{1}{2}CH_3COOC_2H_5$:

| | C(%) | H(%) | N(%) | S(%) |
|---|---|---|---|---|
| Calculated | 68.85 | 5.74 | 5.74 | 6.56 |
| Found | 68.31 | 5.45 | 5.72 | 6.25 |

(ii) Infrared absorption spectra ($cm^{-1}$): 1725 (ester), 3325 (OH).

(iii) Nuclear magnetic resonance spectra ($CDCl_3$):

ppm: 7.28 ( 15 H $(C_6H_5)_3C-$ ),

6.48 ( 1 H OH ),

6.12 ( 1 H 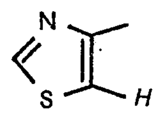 ),

3.66 ( 3 H $-COOCH_3$ ),

3.87 − 3.4 ( 3 H −CH− ).
| 
$CH_2O-$

Reference Example 2

In 50 ml of dioxane was dissolved 2.0 g of the product obtained in Reference Example 1 and after adding thereto 5 ml of a 2N sodium hydroxide solution, the mixture was heated. After stirring the mixture for 30 minutes at 45°C., the solvent was distilled off under reduced pressure and the residue was acidified with hydrochloric acid and extracted with ethyl acetate. The organic layer was collected, washed with water and dried by anhydrous magnesium sulfate. The solvent was distilled off under reduced pressure and then acetonitrile was added to the residue to provide 700 mg of 3-hydroxy-2-(2-tritylaminothiazol-4-yl)propionic acid crystals.

(i) Infrared absorption spectra ($cm^{-1}$): 1710 (—COOH).

(ii) Nuclear magnetic resonance spectra ($d_6$—DMSO):

ppm: 6.28 ( 1 H 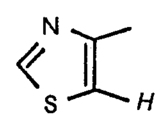 ),

3.4 ( 3 H −CH− )
| 
$CH_2O$

Example 1

In 20 ml of tetrahydrofuran was dissolved 430 mg of 3-hydroxy-2-(2-tritylaminothiazol-4-yl)-propionic acid and the solution was mixed with a solution prepared by dissolving 494 mg of 7β-amino-3-(1-methyltetrazol-5-yl)thiomethyl-$\Delta^3$-cephem-4-carboxylic acid benzhydryl ester in 30 ml of dichloromethane. To the mixed solution was added 206 mg of N,N'-dicyclohexylcarbodiimide and the resultant mixture was stirred for one hour. After distilling off the solvent from the reaction mixture under reduced pressure, 50 ml of dichloromethane was added to the residue and insoluble N,N'-dicyclohexylurea was filtered off. The filtrate was washed successively with acid, an aqueous sodium hydrogencarbonate solution, and water. The filtrate was dried by anhydrous magnesium sulfate, the residue was distilled off under reduced pressure, and after adding 30 ml of ether to the residue formed, the mixture was stirred for one hour. The powder formed was recovered by filtration and washed with ether to provide 680 mg of 7$\beta$-[3-hydroxy-2-(2-tritylaminothiazol-4-yl)propionamido]-3-(1-methyltetrazol-5-yl)thiomethyl-$\Delta^3$-cephem-4-carboxylic acid benzhydryl ester.

(i) Infrared absorption spectra (cm$^{-1}$, KBr): 1770 (lactum), 1710 (ester), 1660 (amide).

(ii) Nuclear magnetic resonance spectra ($d_6$—DMSO)

ppm: 6.10 ( 1 H 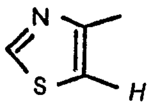 ),

5.67 ( 1 H 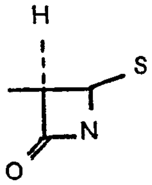 ),

5.06 ( 1 H 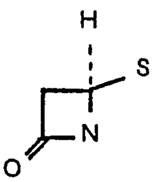 ),

4.24 ( 2 H 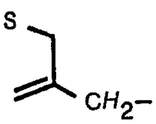 ),

3.83 ( 3 H 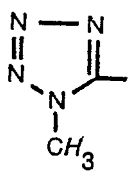 ),

3.7 ( 2 H 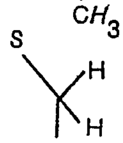 )

3.5 ( 3 H 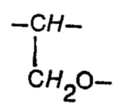 )

Example 2

In 20 ml of tetrahydrofuran was dissolved 430 mg of 3-hydroxy-2-(2-tritylaminothiazol-4-yl)propionic acid and the solution was mixed with a solution prepared by dissolving 494 mg of 7$\beta$-amino-3-(1-methyltetrazol-5-yl)thiomethyl-$\Delta^3$-cephem-4-carboxylic acid benzhydryl ester in 30 ml of dichloromethane. To the mixed solution was added 206 mg of N,N'-dicyclohexylcarbodiimide and the resultant mixture was stirred for one hour. After distilling off the solvent from the reaction mixture under reduced pressure, 50 ml of dichloromethane was added to the residue and insoluble N,N'-dicyclohexylurea was filtered off. The filtrate was washed successively with acid, an aqueous sodium hydrogencarbonate solution, and water. The filtrate was dried by anhydrous magnesium sulfate, the residue was distilled off under reduced pressure, and after adding 30 ml of ether to the residue formed, the mixture was stirred for one hour. The powder formed was recovered by filtration and washed with ether to provide 680 mg of 7$\beta$-[3-hydroxy-2-(2-tritylaminothiazol-4-yl)propionamido]-3-(1-methyltetrazol-5-yl)thiomethyl-$\Delta^3$-cephem-4-carboxylic acid benzhydryl ester.

In 10 ml of dichloromethane was dissolved the product thus obtained and after adding thereto 5 ml of trifluoroacetic acid and 1 ml of anisole, the mixture was stirred for one hour at room temperature. The reaction mixture was dried into solid under reduced pressure and ether was added thereto to give an incitement to the residue to form a powder, which was recovered by filtration. The powder was further added to 10 ml of 10% formic acid followed by stirring for 30 minutes at 55°C. The reaction mixture obtained was dried in a solid under reduced pressure and the residue was washed with isopropyl ether and then purified by column chromatography using Diaion HP 20 (trade name of ionexchange resin, made by Mitsubishi Chemical Industries, Ltd.) to provide 7β-[2-(2-aminothiazol-4-yl)-3-hydroxypropionamido]-3-[(1-methyltetrazol-5-yl)thiomethyl]-$\Delta^3$-cephem-4-carboxylic acid.

(i) Infrared absorption spectra ($cm^{-1}$, KBr): 1760 (lactum), 1660 (amide).

(ii) Nuclear magnetic resonance spectra ($d_6$—DMSO)

ppm: 3.62 ( 2 H 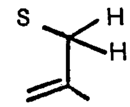 ),

3.68 ( 3 H 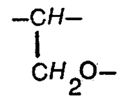 )

3.91 ( 3 H 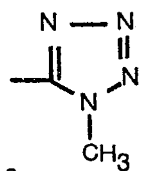 ),

4.28 ( 2 H 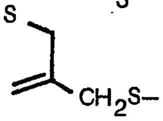 ),

5.0 ( 1 H 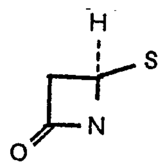 ),

5.63 ( 1 H 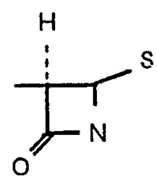 ),

6.19 ( 1 H 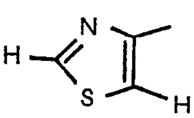 ).

Example 3

In 10 ml of tetrahydrofuran was dissolved 295 mg of 3-hydroxy-2-(2-tritylaminothiazol-4-yl)-propionic acid and after adding thereto 300 mg of 7β-aminocephalosporanic acid benzhydroyl ester and further 141 mg of N,N'-dicyclohexylcarbodiimide, the mixture was stirred for 2 hours at room temperature. Then the solvent was distilled off under reduced pressure and after adding 50 ml of dichloromethane to the residue, insoluble N,N'-dicyclohexylurea was filtered off. The filtrate was washed twice with water acidified by hydrochloric acid, once with an aqueous solution of sodium hydrogencarbonate, and once with an aqueous solution of sodium chloride. After drying the filtrate by anhydrous magnesium sulfate, the solvent was distilled off and ether was added to the residue to provide 440 mg of 3-acetoxymethyl-7β-[3-hydroxy-2-(2-tritylaminothiazol-4-yl)propionamido]-$\Delta^3$-cephem-4-carboxylic acid benzhydryl ester.

The ester was treated as in Example 2 to release the protective group and purified as in Example 2 to provide 3-acetoxymethyl-7β-[2-(2-aminothiazol-4-yl)-3-hydroxypropionamido]-$\Delta^3$-cephem-4-carboxylic acid.

(i) Nuclear magnetic resonance spectra ($d_6$—DMSO)

ppm: 6.43 ( 1 H 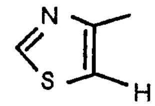 )

5.63 ( 1 H 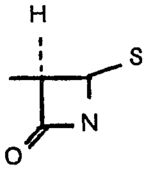 )

5.06 ( 1 H 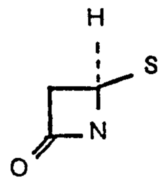 )

4.80 ( 2 H 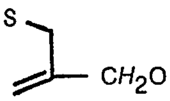 )

3.74 ( 3 H 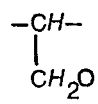 )

3.54 ( 2 H 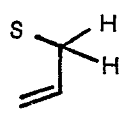 )

2.0 ( 3 H $-OCOCH_3$ )

Example 4

In 20 ml of anhydrous tetrahydrofuran were dissolved 430 mg (1 millimole) of 3-hydroxy-2-(2-tritylaminothiazol-4-yl)propionic acid, 510 mg (1 millimole) of 7β-amino-3-(5-methyl-1,3,4-thiadiazol-2-yl)thiomethyl-$\Delta^3$-cephem-4-carboxylic acid benzhydryl ester, and after adding thereto 206 mg (1 millimole) of N,N'-dicyclohexylcarbodiimide, the mixture was stirred for 3 hours at room temperature. The reaction mixture was concentrated under reduced pressure and then 40 ml of ethyl acetate was added to the residue. Insoluble N,N'-dicyclohexylurea formed was filtered off and the filtrate was washed with a dilute aqueous acid solution and an aqueous solution of sodium hydrogencarbonate and dried by anhydrous magnesium sulfate. The solvent was distilled off under reduced pressure and then the residue was treated with ether to form a powder, which was recovered by filtration and washed with water to provide 720 mg of 7β-[3-hydroxy-2-(2-tritylaminothiazol-4-yl)propionamido]-3-(5-methyl-1,3,4-thiadiazol-2-yl)thiomethyl-$\Delta^3$-cephem-3-carboxylic acid benzhydryl ester.

The product was dissolved in a mixture of 15 ml of trifluoroacetic acid and 1 ml of anisole followed by stirring for 10 minutes at room temperature. After further adding 10 ml of water to the mixture at temperatures below room temperature, the resultant mixture was stirred for 30 minutes at room temperature.

The reaction mixture was concentrated under reduced pressure and ether was added to the residue and the wall of the container was rubbed to form a powder, which was recovered by filtration and washed with ether. The powder was suspended in 10 ml of isopropanol and after adding pyridine to the suspension and adjusting the pH to 4, the mixture was stirred for 30 minutes at room temperature. The powder was then recovered by filtration and washed with isopropanol to provide crude 7β-[2-(2-aminothiazol-4-yl)-3-hydroxypropionamido]-3-(5-methyl-1,3,4-thiadiazol-2-yl)thiomethyl-$\Delta^3$-cephem-4-carboxylic acid. The product was purified by column chromatography using Diaion HP 20 (trade name) to provide a pure product.

(i) Infrared absorption spectrum ($cm^{-1}$, KBr): 1770 (lactam).

(ii) Nuclear magnetic resonance spectra ($D_6$—DMSO)

ppm: 2.65 ( 3 H 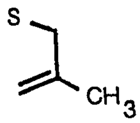 )

3.74 ( 5 H 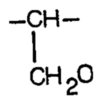 , 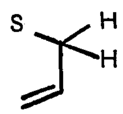 ),

4.34 ( 2 H 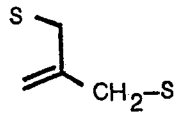 ,

5.07 ( 1 H 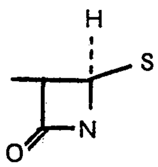 ),

5.64 ( 1 H 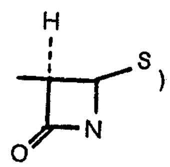 ),

6.45 ( 1 H 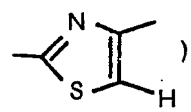 ).

Example 5

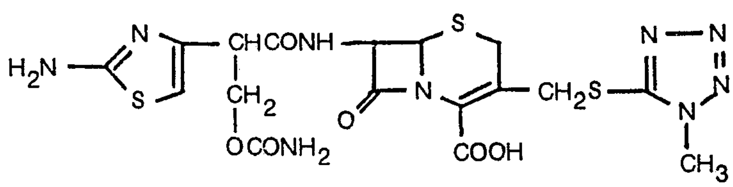

In 20 ml of dichloromethane was dissolved benzhydryl 7β-[3-hydroxy-2-(2-tritylaminothiazol-4-yl)propionamido]-3-(1-methyltetrazol-5-yl)thiomethyl-$\Delta^3$-cephem-4-carboxylate and then 0.2 ml of trichloroacetyl isocyanate was added to the solution at 10°C. to cause reaction for 20 minutes. The reaction mixture was washed with water and dried by anhydrous magnesium sulfate. The solvent was distilled off, the residue was subjected to column chromatography using 30 g of silicagel and the product was eluted with a mixture of benzene and ethyl acetate in a 3:1 by volume ratio. The required fractions were collected and concentrated to provide 400 mg of 3-(1-methyltetrazol-5-yl)thiomethyl-7β-[3-trichloroacetylcarbamoyloxy-2-(2-tritylaminothiazol-4-yl)propionamido]-$\Delta^3$-cephem-4-carboxylic acid benzhydryl ester.

(i) Infrared absorption spectra ($cm^{-1}$, KBr): 1780, 1715, 1670.

(ii) Nuclear magnetic resonance spectra ($CDCl_3$)

ppm: 6.91 ( 1 H 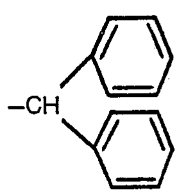 ),

6.17 ( 1 H 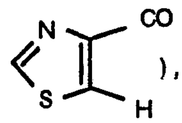 ),

5.80 ( 1 H 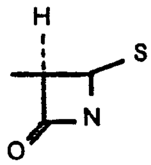 ),

4.96 ( 1 H 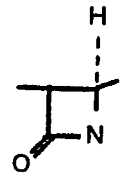 ).

By treating the N-trichloroacetylcarbamoyl compound in conventional manner to release the protective group, 7$\beta$-[2-(2-aminothiazol-4-yl)-3-carbamoyloxypropionamido]-3-(1-methyltetrazol-5-yl)thiomethyl-$\Delta^3$-cephem-4-carboxylic acid was obtained.

(i) Infrared absorption spectra ($cm^{-1}$, KBr): 1775, 1660.

(ii) Nuclear magnetic resonance spectra ($d_6$—DMSO)

ppm: 6.50 ( 1 H 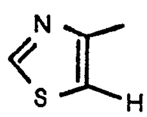 ),

5.63 ( 1 H 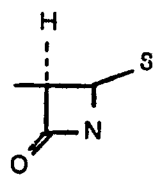 ),

5.03 ( 1 H 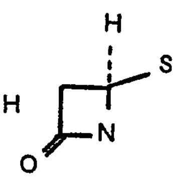 ).

Example 8

| | |
|---|---|
| Product of Example 2 | 250 g |
| Corn starch | 50 g |
| Talc | 5 g |
| Silicic anhydride | 1.5 g |

The above mixture was finely pulverized and filled in 1,000 capsules.

## Claims

1. A cephalosporin derivative represented by the general formula

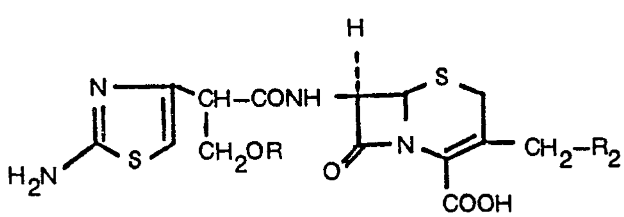

wherein

R represents hydrogen or a carbamoyl group and $R_2$ represents an acetoxy group, a (1-methyltetrazol-5-yl)thio group or a (5-methyl-1,3,4-thiadiazol-2-yl)thio group, and optionally one or both of the amino group and the carboxylic acid group is protected.

2. 7β-[2-(2-Aminothiazol-4-yl)-3-hydroxypropionamido]-3-[(1-methyltetrazol-5-yl)thiomethyl]-$\Delta^3$-cephem-4-carboxylic acid according to claim 1.

3. 3-Acetoxymethyl-7β-[2-(2-aminothiazol-4-yl)-3-hydroxypropionamido]-$\Delta^3$-cephem-4-carboxylic acid according to claim 1.

4. 7β-[2-(2-Aminothiazol-4-yl)-3-hydroxypropionamido]-3-(5-methyl-1,3,4-thiadiazol-2-yl)thiomethyl-$\Delta^3$-cephem-4-carboxylic acid according to claim 1.

5. 7β-[2-(2-aminothiazol-4-yl)-3-carbamoyloxypropionamido]-3-(1-methyltetrazol-5-yl)thiomethyl-$\Delta^3$-cephem-4-carboxylic acid according to claim 1.

6. A salt of a compound according to any preceding claim.

7. A process of producing a cephalosporin derivative according to claim 1 which comprises reacting (a) a 7-amino cephalosporin derivative represented by the general formula

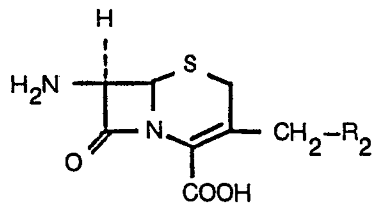

or a reactive derivative thereof in which the amino group is substituted, wherein $R_2$ has the same significance as in claim 1 and the carboxy group may be protected by a conventional protective group, with (b) a thiazolylpropionic acid derivative represented by the general formula

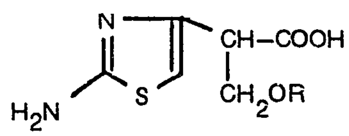

or a reactive derivative thereof in which the carboxylic acid group is substituted, wherein R has the same significance as in claim 1 and the hydroxy group when R is hydrogen and/or the amino group may be protected by a conventional protective group, if desired treating the product to convert any protected group to the free group, and optionally converting the reaction product to a salt thereof.

8. A pharmaceutical composition comprising a cephalosporin derivative according to any of claims 1 to 6 in a pharmaceutically acceptable carrier.

**Patentansprüche**

1. Ein Cephalosporinderivat der allgemeinen Formel

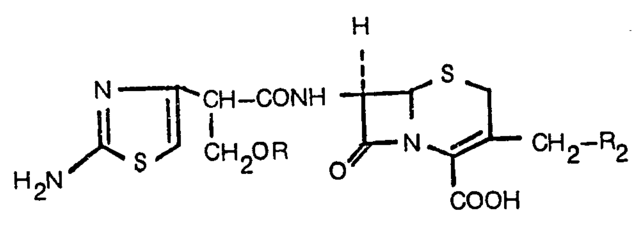

in der R Wasserstoff oder eine Carbamoylgruppe und $R^2$ eine Acetoxygruppe, eine (1-Methyl-tetrazol-5-yl)thiogruppe oder eine (5-Methyl-1,3,4-thiadiazol-2-yl)thiogruppe bedeutet und gegebenenfalls eine der beiden Aminogruppen oder beide Aminogruppen und die Carbonsäuregruppe geschützt sind.

2. 7β-[2-(2-Aminothiazol-4-yl)-3-hydroxypropionamido]-3-[(1-methyltetrazol-5-yl)thiomethyl]-$\Delta^3$-cephem-4-carbonsäure gemäß Anspruch 1.

3. 3-Acetoxy-7β-[2-(2-aminothiazol-4-yl)-3-hydroxypropionamido]-$\Delta^3$-cephem-4-carbonsäure gemäß Anspruch 1.

4. 7β-[2-(2-Aminothiazol-4-yl)-3-hydroxypropionamido]-3-(5-methyl-1,3,4-thiadiazol-2-yl)thiomethyl-$\Delta^3$-cephem-4-carbonsäure gemäß Anspruch 1.

5. 7β-[2-(2-Aminothiazol-4-yl)-3-carbamoyloxypropionamido]-3-(1-methyltetrazol-5-yl)thiomethyl-$\Delta^3$-cephem-4-carbonsäure gemäß Anspruch 1.

6. Ein Salz einer Verbindung nach einem der vorangegangenen Ansprüche.

7. Verfahren zur Herstellung eines Cephalosporinderivats nach Anspruch 1, dadurch gekennzeichnet, daß man (a) ein 7-Aminocephalosporinderivat der allgemeinen Formel

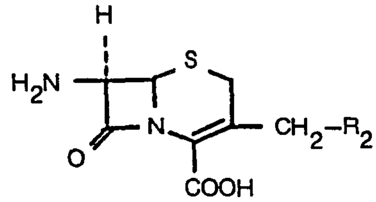

oder ein reaktionsfähiges Derivat davon, dessen Aminogruppe substituiert ist, wobei $R^2$ die gleiche Bedeutung wie in Anspruch 1 hat und die Carboxygruppe durch eine gebräuchliche Schutzgruppe geschützt sein kann, mit (b) einem Thiazolylpropionsäurederivat der allgemeinen Formel

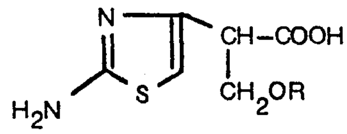

oder einem reaktionsfähigen Derivat davon, in dem die Carbonsäuregruppe substituiert ist, wobei R die gleiche Bedeutung hat wie in Anspruch 1 und die Hydroxygruppe, wenn R für Wasserstoff und/oder die Aminogruppe steht, durch eine übliche Schutzgruppe geschützt sein kann, umsetzt, gegebenenfalls die erhaltene Verbindung behandelt, um etwa vorhandene Schutzgruppen in die freien Gruppen umzuwandeln und gegebenenfalls das Reaktions-produkt in ein Salz davon umwandelt.

8. Arnzneimittel, enthaltend ein Cephalosporinderivat nach einem der Ansprüche 1 bis 6, in einem pharmazeutisch verträglichen Träger.

**Revendications**

1. Un dérivé de la céphalosporine représenté par la formule générale

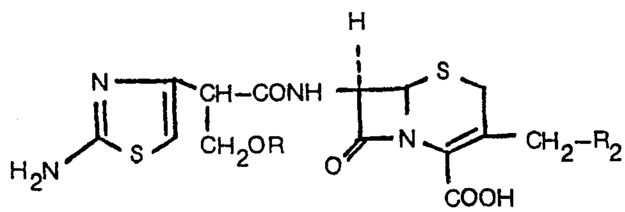

dans laquelle R représente un hydrogène ou un groupe carbamoyle et $R_2$ représente un groupe acétoxy, un groupe (1-méthyltétrazol-5-yl)thio ou un groupe (5-méthyl-1,3,4-thiadiazol-2-yl)thio et optionnellement l'un ou les deux parmi le groupe amino et le groupe acide carboxylique est ou sont protégés.

2. Le dérivé selon la revendication 1 constitué par l'acide 7$\beta$-[2-(2-aminothiazol-4-yl)-3-hydroxypropionamido]-3-[(1-méthyltétrazol-5-yl)thiométhyl]-$\Delta^3$-céphem-4-carboxylique.

3. Le dérivé selon la revendication 1 constitué par l'acide 3-acétoxyméthyl-7$\beta$-[2-(2-aminothiazol-4-yl)-3-hydroxypropionamido]-$\Delta^3$-céphem-4-carboxylique.

4. Le dérivé selon la revendication 1 constitué par l'acide 7$\beta$-[2-(2-aminothiazol-4-yl)-3-hydroxypropionamido]-3-(5-méthyl-1,3,4-thiadiazol-2-yl)thiométhyl-$\Delta^3$-céphem-4-carboxylique.

5. Le dérivé selon la revendication 1 constitué par l'acide 7$\beta$-[2-(2-aminothiazol-4-yl)-3-carbamoyloxypropionamido]-3-(1-méthyltétrazol-5-yl)thiométhyl-$\Delta^3$-céphem-4-carboxylique.

6. Le sel d'un composé selon l'une quelconque des revendications précédentes.

7. Un procédé de production d'un dérivé de la céphalosporine selon la revendication 1 qui consiste à faire réagir (a) un dérivé de 7-amino-céphalosporine représenté par la formule générale

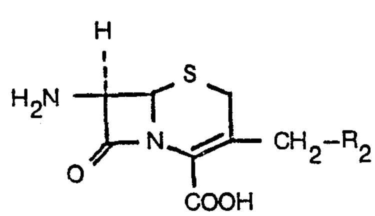

ou un dérivé réactif de celle-ci dans lequel le groupe amino est substitué, dans laquelle $R_2$ a la même signification que dans la revendication 1 et le groupe carboxy peut être protégé par un groupe protecteur conventionnel avec (b) un dérivé de l'acide thiazolylpropionique représenté par la formule générale

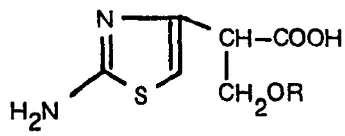

ou un dérivé réactif de celui-ci dans lequel le groupe acide carboxylique est substitué, dans laquelle R a la même signification que dans la revendication 1 et le groupe hydroxy lorsque R est un hydrogène et/ou le groupe amino peuvent être protégés par un groupe protecteur conventionnel, en traitant, si on le désire, le produit pour convertir le groupe protégé quelconque en le groupe libre et en convertissant optionnellement le produit de la réaction en un de ses sels.

8. Une composition pharmaceutique constituée par un dérivé de la céphalosporine selon l'une quelconque des revendications 1 à 6 dans tout support acceptable pharmaceutiquement.